# EUROPEAN PATENT APPLICATION

(11) **EP 2 116 231 A1**
(43) Date of publication of application: **11.11.2009**
(21) Application number: 08008614.3
(22) Date of filing: 07.05.2008
(51) Int. Cl.: A61K 9/16, A61K 9/28

(54) **Granulate comprising escitalopram oxalate**

(71) Applicant: Hexal AG, 83607 Holzkirchen (DE)
(72) Inventor: Lipsdorf, Antje, 83607 Holzkirchen (DE); Neumüller, Alexandra, 81543 München (DE); Wolf, Claudia, 85591 Vaterstetten (DE)
(74) Representative: Forstmeyer, Dietmar

(57) **Abstract**

The invention relates to a granulate comprising escitalopram oxalate having an average particle size of less than 100 µm and at least one filler. The invention further relates to a method of producing that granulate and also to a tablet or capsule comprising that granulate and/or obtained by compressing that granulate to form a tablet or by filling that granulate into a capsule shell. Finally, the invention relates to the use of that granulate and/or tablet or capsule in the treatment or prevention of a disorder from the spectrum of depressive disorders.

## Description

### FIELD OF THE INVENTION

The invention relates to a granulate comprising escitalopram oxalate having a particular, small average particle size and at least one filler. The invention further relates to a method of producing that granulate and also to a tablet or capsule comprising that granulate and/or obtained by compressing that granulate to form a tablet or by filling that granulate into a capsule shell. Finally, the invention relates to the use of that granulate and/or tablet or capsule in the treatment or prevention of a disorder from the spectrum of depressive disorders.

### PRIOR ART

Citalopram, 1-[3-(dimethylamino)propyl]-1-(4-fluorophenyl)-1,3-dihydroisobenzofuran-5-carbonitrile, was first disclosed in DE 2 657 013 and US 4,136,193. It belongs to the class of selective serotonin reuptake inhibitors (SSRIs), which selectively inhibit the reuptake of serotonin into nerve cells from the synaptic cleft. Citalopram is therefore mainly used as an antidepressant and as an anxiolytic. The average daily dose is 20 mg. Citalopram is marketed in the form of the hydrobromide salts and hydrochloride salts.

The preparation of the (S) enantiomer of citalopram is described in US 4,943,590. It is referred to as escitalopram, (S)-1-[3-(dimethylamino)propyl]-1-(4-fluorophenyl)-1,3-dihydroisobenzofuran-5-carbonitrile. Escitalopram is the pharmacologically active enantiomer of citalopram. As a result of using escitalopram instead of citalopram, the dose required for the pharmacological effect can be substantially reduced. Not only is (R)-citalopram without antidepressant activity but there is even discussion that rather it impedes the action of escitalopram (C. Sanchez, K.P. Bogeso, B. Ebert, E.H. Reines, C. Braestrup, Psychopharmacology 174 (2004), 163-176; N. Moore, H. Verdoux and B. Fantino, Int. Clin. Psychopharmacol. 20 (2005) 131-137; M.E. Mansari, O. Wiborg, O. Mnie-Filali, N. Benturquia, C. Sanchez, N. Haddjeri, Int. J. Neuropsychopharmacol. 10 (2007) 31-40).

As a result of the use of escitalopram instead of citalopram it is accordingly possible not only for the dose required for the pharmacological effect to be substantially reduced but also for the undesirable side-effects caused by (R)-citalopram to be avoided. Like citalopram, escitalopram is mainly used as an antidepressant and as an anxiolytic. The average daily dose is 10 mg. Escitalopram is marketed in the form of the oxalate salt.

In WO 03/011278 A1 there is described as a pharmaceutical dosage form a tablet which was obtained by direct compression of escitalopram oxalate together with customary pharmaceutical excipients. Because escitalopram oxalate can undergo direct compression only from a particular average particle size upwards, there were used in the course thereof crystalline particles of escitalopram oxalate having an average particle size of at least 40 µm, preferably from 50 to 200 µm, and in the disclosed example 163 µm. These particles were obtained by means of a specific crystallisation process, which is also described in WO 03/011278 A1.

However, the fact that, as mentioned hereinbefore, escitalopram has to be administered only in a relatively small dose, especially in a substantially smaller dose than citalopram, gives rise to a problem, when using such large crystalline particles of escitalopram oxalate as are described in WO 03/011278 A1, that it is no longer possible for these large active ingredient particles to be distributed as homogeneously and as uniformly as desired in the tablet or in the material to be compressed to form tablets and accordingly, in addition to a less reliable active ingredient content in the individual tablet, they also have a disadvantageous effect on the bioavailability of escitalopram.

When escitalopram oxalate is used in the form of large crystalline particles, the bioavailability is impaired especially as a result of the fact that the dissolution and, therefore, the release of the active ingredient is delayed, which can result in slower active ingredient uptake in the body and, therefore, in a later onset of the desired pharmacological effect. This is also relevant to escitalopram, notwithstanding the fact that escitalopram, like all SSRIs, develops its antidepressant action fully only after some weeks, because the action of escitalopram, especially its anxiolytic action, starts quickly after absorption into the body. In addition, large crystalline particles of the active ingredient can result in the crystals not dissolving completely and therefore in the active ingredient not being released in its entirety but rather in portions of the large crystals passing through the gastro-intestinal tract unchanged, so that the exact active ingredient dosage becomes unreliable.

The present inventors have discovered that these problems of non-uniform active ingredient distribution and of impaired bioavailability can be avoided by using small and/or fine active ingredient particles which, by means of granulation, are brought into a state in which they can be compressed to form a tablet or filled into a capsule shell.

However, a problem arises when granulating particles of pharmaceutical substances that during the granulation, as a result of the effects of oxygen, heat and/or moisture, there may be formed degradation products of the pharmaceutical substance which, in addition to reducing the content of the active substance, also result in undesirable impurities in the pharmaceutical product and also in reduced storage stability of the pharmaceutical product.

### PROBLEM OF THE INVENTION

The problem of the present invention is to develop a pharmaceutical dosage form for escitalopram oxalate in which both the active ingredient is homogeneously distributed and therefore has greater bioavailability and also impurities arising in the course of formulation are minimised.

### DETAILED DESCRIPTION OF THE INVENTION

The present invention relates to a granulate which comprises escitalopram oxalate having an average particle size of less than 100 µm and at least one filler.

The active ingredient designation "escitalopram oxalate" includes all compounds which include both escitalopram and also oxalate, especially salts having an equimolar ratio of escitalopram and oxalate, and also all hydrates, solvates and polymorphic forms thereof.

The term "average particle size", also referred to as average grain size or average grade size, describes the median of the particle size distribution, which distribution can be determined by means of laser diffraction at 1 bar dispersive pressure using, for example, a Sympatec Helos apparatus.

The escitalopram oxalate used has an average particle size of less than 100 µm, especially greater than 0.5 µm and less than 100 µm, preferably from 0.6 µm to 60 µm, more preferably from 0.6 µm to 40 µm, even more preferably from 0.6 µm to less than 40 µm, even more preferably from 0.6 µm to 30 µm, even more preferably from 0.6 µm to 20 µm, even more preferably from 0.6 µm to less than 20 µm, even more preferably from 0.7 µm to 15 µm, most preferably from 0.8 µm to 10 µm.

The inventors have established that, by appropriately selecting the filler in the material to be granulated, the formation of active ingredient degradation products during the granulation procedure and accordingly the presence of impurities in the granulate can be reduced. Especially suitable fillers include sugars and sugar alcohols such as, for example, lactose, sucrose, glucose and mannitol, macromolecular fillers such as, for example, starches, especially maize starch, rice starch, potato starch and wheat starch, and also inorganic fillers such as, for example, calcium phosphates.

The particle size of the filler should not be too large to ensure adequate homogeneity in the granulate according to the invention. The average particle size of the filler is preferably not greater than 200 µm.

Special preference is given to using lactose as filler, whilst microcrystalline cellulose has been found to be less suitable as filler and accordingly in a particular embodiment of the present invention the granulate contains less than 10 % by weight microcrystalline cellulose, preferably less than 5 % by weight microcrystalline cellulose, more preferably less than 2 % by weight microcrystalline cellulose, even more preferably less than 1 % by weight microcrystalline cellulose, in each case based on the filler, and most preferably no microcrystalline cellulose.

Mixtures of the above-mentioned fillers may also be used. Preferably, at least one constituent of the filler mixtures is lactose. Further preference is given to the filler containing at least 50 % by weight lactose, more preferably at least 60 % by weight lactose, even more preferably at least 70 % by weight lactose, even more preferably at least 80 % by weight lactose and most preferably at least 90 % by weight lactose, in each case based on the filler.

The present invention relates also to methods of producing a granulate as described hereinbefore. In this context, the above definitions and explanations also apply.

The granulate of the present invention can be obtained using various granulating techniques. These include both wet granulation and also dry granulation.

In a preferred embodiment, wet granulation is used. This includes both paste granulation, with use of a binder, and also so called "crust granulation" (german: "Krustengranulierung") or "binderless granulation", with use of solvents or solvent vapours.

In a preferred embodiment, wet granulation is employed with use of a binder. In that context it has been found, surprisingly, that it is the selection of filler rather than the selection of binder that is decisive for avoiding the formation of active ingredient degradation products during the granulation procedure.

As binder there may be used therefore any binder customary in the pharmaceutical sector, including, *inter alia*, sugars, especially sucrose, macromolecular binders such as, for example, starches, especially maize starch, rice starch, potato starch and wheat starch, gelatin, tragacanth, cellulose ethers, especially hydroxypropylcellulose, hydroxypropyl methylcellulose (HMPC) and carboxymethylcellulose sodium, and polyvinylpyrrolidones, also referred to as polyvidones or povidones, especially copovidones. Preference is given to using HPMC or copovidones as binder. The binder is contained in the granulate according to the invention preferably in an amount of from 2 to 10 % by weight.

It has been found to be advantageous for avoiding the formation of active ingredient degradation products if during granulation less than 10 % by weight microcrystalline cellulose, preferably less than 5 % by weight microcrystalline cellulose, more preferably less than 2 % by weight microcrystalline cellulose, even more preferably less than 1 % by weight microcrystalline cellulose, in each case based on the total amount of the filler, and most preferably no microcrystalline cellulose is present in the material being granulated.

The granulation is preferably carried out as wet granulation using a binder with subsequent fluidised-bed drying. The wet granulation is preferably carried out in a mixing granulator (high-speed mixer). For this purpose, the active ingredient is pre-mixed with the filler or fillers in the granulator and subsequently granulated using the binder solution. Alternatively, the binder may also be added to the pre-mix in dry powder form and granulation carried out using only the liquid (for example, water).

The present invention relates also to pharmaceutical dosage forms for the granulate according to the invention. Pharmaceutical dosage forms according to the invention that come into consideration are, especially, tablets and capsules. However, use of the granulate according to the invention in the form of a powder for oral suspension, or for the purpose of producing a solution, especially a solution for injection or a solution for infusion, is also possible. The granulate according to the invention can itself also be used directly as a pharmaceutical dosage form according to the invention.

The pharmaceutical dosage forms according to the invention may furthermore comprise further customary pharmaceutical excipients, especially disintegrants or disintegration accelerators, dry binders, flow regulators, lubricants, antisticking agents, film-forming agents, plasticisers and/or pigments.

Any disintegrant customary in the pharmaceutical sector may be used as a disintegrant. The disintegrants are added preferably after granulation and are then a constituent of the external phase of the granulate for improving dissolution of the granulate in the body. They include, *inter alia*, starches and starch derivatives, especially maize starch, rice starch, potato starch and wheat starch, polyvinylpyrrolidone, especially cross-linked polyvinylpyrrolidone, and carboxymethylcellulose sodium or croscarmellose sodium, especially low-substituted carboxymethylcellulose sodium. Preference is given to the use of croscarmellose sodium as disintegrant. The disintegrant is contained in the granulate according to the invention preferably in an amount of from 3 to 6 % by weight.

Any dry binder customary in the pharmaceutical sector may be used as a dry binder. The dry binders are added preferably after granulation and are then a constituent of the external phase of the granulate for improving the compressibility of the granulate. They include, *inter alia*, mannitol, cellulose, especially microcrystalline cellulose, and calcium phosphates. Preference is given to the use of microcrystalline cellulose as dry binder.

Any flow regulator customary in the pharmaceutical sector may be used as a flow regulator. The flow regulators are added preferably after granulation and are then a constituent of the external phase of the granulate for optimising the flow properties of the granulate. Preference is given to the use of colloidal silica as flow regulator. The flow regulator is contained in the granulate according to the invention preferably in an amount of from 0.1 to 2 % by weight.

Any lubricant customary in the pharmaceutical sector may be used as a lubricant. The lubricants are added preferably after granulation and are then a constituent of the external phase of the granulate for reducing the friction between the granulate being tabletted and the tabletting tool. They include, *inter alia*, fatty acids, especially stearic acid, salts of fatty acids with divalent metals, especially magnesium stearate, calcium arachinate, fatty alcohols, especially stearyl alcohol, sodium stearyl fumarate, polyethylene glycols and talc. Preference is given to the use of magnesium stearate as lubricant. The lubricant is contained in the granulate according to the invention preferably in an amount of from 0.1 to 5 % by weight.

Any film-forming agent customary in the pharmaceutical sector may be used as a film-forming agent. Film-forming agents include, *inter alia*, cellulose derivatives, especially methylcellulose, hydroxypropyl methylcellulose, also referred to as hypromellose, hydroxypropylcellulose, hydroxyethylcellulose, carboxymethylcellulose sodium, ethylcellulose, cellulose acetate phthalate and hydroxypropyl methylcellulose phthalate, polyacrylates and polymethacrylates, especially Eudragit E, NE, L, S, RL and RS, vinyl polymers, especially polyvinylpyrrolidone and polyvinyl acetate phthalate, and natural film-forming agents, especially shellac. Preference is given to the use of hypromellose as film-forming agent.

Any antisticking agent customary in the pharmaceutical sector may be used as an antisticking agent. The antisticking agents are added preferably during film-forming and are then a constituent of the film for preventing the tablets from adhering to one another during coating. Preference is given to the use of talc as antisticking agent.

Any plasticiser customary in the pharmaceutical sector may be used as a plasticiser. The plasticisers are added preferably during film-forming and are then a constituent of the film. Preference is given to the use of polyethylene glycols, also referred to as macrogols, as plasticiser.

Any pigment customary in the pharmaceutical sector may be used as a pigment. The pigments are added preferably during film-forming and are then a constituent of the film. Preference is given to the use of titanium oxide as pigment.

The pharmaceutical dosage forms according to the invention, especially tablets or capsules according to the invention, comprise the granulate according to the invention. A tablet according to the invention can be obtained especially by compressing a granulate according to the invention to form the tablet. A capsule according to the invention can be obtained by filling a granulate according to the invention into a capsule shell.

As tablets there can be produced simple tablets, lozenges, chewable tablets, sublingual tablets, effervescent tablets, dispersible tablets, multilayer tablets, enteric-coated tablets or matrix tablets. Compression to form tablets can be carried out by the customary methods for tablet compression, especially using eccentric presses or rotary presses. Special preference is given to the production of film-coated tablets.

As capsules there can be produced hard gelatin capsules. Filling of the capsule shells can be carried out by the customary methods for capsule filling.

The amount of escitalopram oxalate used in the pharmaceutical dosage form according to the invention is governed by the desired pharmacological effects and furthermore depends especially on the age, sex and weight of the person to be treated. Usual amounts of escitalopram oxalate in the pharmaceutical dosage form according to the invention are, for example, 2.5 mg, 5 mg, 10 mg or 20 mg, in each case based on escitalopram.

The present invention relates also to the use of a pharmaceutical dosage form according to the invention, especially a granulate according to the invention or a tablet or capsule according to the invention, in the treatment or prevention of a disorder from the spectrum of depressive disorders, especially in patients with inhibited-depressive syndrome. Use in the treatment or prevention of panic disorders and anxiety disorders, especially social phobia and agoraphobia, is also possible with a pharmaceutical dosage form according to the invention.

### Examples

Escitalopram oxalate was used in the following examples of particle size distribution:

| | I | II |
|---|---|---|
| D(0.9) | 37 µm | 7 µm |
| D(0.5) | 18 µm | 2 µm |
| D(0.1) | 5 µm | 1 µm |

The values of D(0.1), D(0.5) and D(0.9) denote the particle sizes up to which cumulatively 10 %, 50 % and 90 %, respectively, of particles in the particle size distribution were found to lie. D(0.1) and D(0.9) accordingly correspond to the 0.1-quantile and 0.9-quantile, respectively, of the particle size distribution; D(0.5) corresponds to the median of the particle size distribution.

### Examples of formulations (amounts given in grams):

| | | **Example 1** | **Example 2** |
|---|---|---|---|
| **Material to be granulated** | Escitalopram oxalate | 6.390 | 6.390 |
| | Lactose monohydrate | - | 95.860 |
| | Microcrystalline cellulose (as filler) | 95.860 | - |
| | Copovidone | 3.750 | 3.750 |
| **External phase of granulate** | Microcrystalline cellulose (as dry binder) | 9.625 | 9.625 |
| | Croscarmellose sodium | 6.250 | 6.250 |
| | Colloidal silica | 1.250 | 1.250 |
| | Magnesium stearate | 1.875 | 1.875 |
| **Film** | Hypromellose | 3.450 | 3.450 |
| | Macrogol | 0.850 | 0.850 |
| | Titanium dioxide | 0.450 | 0.450 |
| | Talc | 0.250 | 0.250 |

In Examples 1 and 2, the total impurities formed were measured (i) immediately after production and (ii) after storage for 1 month at 40°C and 75 % atmospheric humidity:

| | Example 1 | Example 2 |
|---|---|---|
| (i) | 0.32 % | 0.08 % |
| (ii) | 0.55 % | 0.15 % |

These results show that, by selecting a suitable filler such as, for example, lactose, the formation of active ingredient degradation products during granulation and consequently the presence of impurities in the pharmaceutical dosage form according to the invention can be substantially reduced. At the same time, the storage stability of the pharmaceutical dosage form according to the invention is increased as a result.

## Claims

1. Granulate, **characterised in that** it comprises escitalopram oxalate having an average particle size of less than 100 µm and at least one filler.

2. Granulate according to claim 1, **characterised in that** the filler comprises at least 50 % by weight lactose, based on the filler.

3. Granulate according to claim 1 or 2, **characterised in that** it additionally comprises at least one binder.

4. Granulate according to one of the preceding claims, **characterised in that** the filler comprises less than 10 % by weight microcrystalline cellulose, based on the filler.

5. Method of producing a granulate, wherein escitalopram oxalate having an average particle size of less than 100 µm is granulated with at least one filler.

6. Method of producing a granulate according to claim 5, wherein the filler comprises at least 50 % by weight lactose, based on the filler.

7. Method of producing a granulate according to claim 5 or 6, wherein the escitalopram oxalate is wet-granulated with at least one filler.

8. Method of producing a granulate according to claim 7, wherein the escitalopram oxalate is wet-granulated with at least one filler and at least one binder.

9. Method of producing a granulate according to one of claims 5 to 8, wherein during granulation less than 10 % by weight microcrystalline cellulose, based on the filler, is contained in the material being granulated.

10. Granulate, producible by a method according to one of claims 5 to 9.

11. Tablet, **characterised in that** it comprises a granulate according to one of claims 1 to 4 or 10.

12. Tablet obtainable by compressing a granulate according to one of claims 1 to 4 or 10 to form the tablet.

13. Capsule, **characterised in that** it comprises a granulate according to one of claims 1 to 4 or 10.

14. Capsule obtainable by filling a granulate according to one of claims 1 to 4 or 10 into a capsule shell.

15. Use of a granulate according to one of claims 1 to 4 or 10 or a tablet according to one of claims 11 or 12 or a capsule according to one of claims 13 or 14 in the treatment or prevention of a disorder from the spectrum of depressive disorders.
